# EUROPÄISCHE PATENTANMELDUNG

(11) **EP 0 556 565 A2**
(43) Veröffentlichungstag der Anmeldung: **25.08.1993**
(21) Anmeldenummer: 93100582.1
(22) Anmeldetag: 15.01.1993
(51) Int. Cl.: A61K 31/22

(54) **Kombinationspräparate von Dipivalylepinephrin mit Carbachol oder Beta-Blockern zur Augeninnendrucksenkung**

(30) Priorität: 17.01.1992 DE 4201079
(62) Teilanmeldung aus: 93116081.6
(71) Anmelder: Gramer, Eugen, Prof.Dr.med.Dr.jur., D-97080 Würzburg (DE)
(72) Erfinder: Gramer, Eugen, Prof.Dr.med.Dr.jur., D-97080 Würzburg (DE)
(74) Vertreter: von Füner, Alexander, Dr.

(57) **Zusammenfassung**

Kombinationspräparat zur Senkung des Augeninnendrucks, bestehend aus:
einer Dipivalylepinephrin-Carbachol-Kombination (Präparat I oder
einer Dipivalylepinephrin-Betablocker-Kombination (Präparat II).

Die Kombinationspräparate können sowohl als Lösung als auch in Suspensionstechnologie hergestellt werden und all diesen Kombinationspräparaten ist gemeinsam, daß sie nur 2 x täglich appliziert werden müssen.

## Beschreibung

Die Erfindung bezieht sich auf ein Kombinationspräparat auf der Basis von DPE zur lokalen Anwendung am Auge zur Senkung des krankhaft erhöhten Augeninnendrucks bei Glaukom. Dipivalylepinephrin (DPE) ist Bestandteil der deutschen Arzneimittel D-Epifrin 0,1 %® und Glaucothil 0,1 %® und Bestandteil der Kombinationspräparate Thiloadren® und Thilodigon 0,5 %® (Patentschrift DE 30 33 898 C2). Das erfindungsgemäße Kombinationspräparat benötigt nur noch 2 Applikationen täglich (morgens und abends) und enthält als zweite Wirkstoffkomponente Carbachol oder einen Betablocker (Beta 1-Beta 2-Blocker oder selektiver Beta-1-Blocker.

### Stand der Technik:

Bisher sind folgende Kombinationspräparate bekannt:
Direktes Parasympathomimetikum/Direktes Sympatholytikum
Pilocarpin 2 % und Metipranolol 0,1 % (Normoglaucon®)
Pilocarpin 2 % und Timolol 0,5 % (Timpilo®)
Direktes Parasympathomimetikum/Direktes Sympathomimetikum
Pilocarpin 1 % und Dipivalylepinephrin 0,1 % (Thiloadren®)
Pilocarpin 2 % und Epinephrin 2 % (Piladren®)
Indirektes Sympatholytikum/Direktes Sympathomimetikum
Guanethidin 0,5 % und Dipivalylepinephrin 0,1 % (Thilodigon® 0,5 %)
Guanethidin 1 % und Adrenalin 0,2 % (Suprexon®)
Guanethidin 3 % und Adrenalin 0,5 % (Suprexon®)
Direktes Parasympathomimetikum/reversibler Cholinesterasehemmer
Pilocarpin 2 % und Neostigmin 1 % (Syncarpin®)
Pilocarpin 2 % und Physostigmin 0,25 % (Isopto-Pilomin®)
Pilocarpin 2 % und Physostigmin 0,5 % (Pilo-Eserin®)
Der Erfindung liegt also die Aufgabe zugrunde, Kombinationspräparate zur Verfügung zu stellen, mit denen im Vergleich zur Gabe der Einzelpräparate eine verstärkte Senkung des Augeninnendrucks erreicht werden kann.

Diese Aufgabe wird wie aus den nachstehenden Ansprüchen ersichtlich gelöst. Dabei ist bei dem erfindungsgemäßen Präparat die Handhabung vereinfacht (nur noch 1 Fläschchen) und so die Gefahr fehlerhafter Anwendung geringer und damit dieses Präparat (Präparat 1) auch bei Glaukom mit engem Kammerwinkel durch das Überwiegen der Miotikawirkung anwendbar, ohne daß die Gefahr eines Glaukomanfalls besteht. Durch diese fixe Kombination werden DPE-Präparate erstmals auch bei engem Kammerwinkel anwendbar. Im Präparat I wird Carbachol, ein direktes Parasympathomimetikum mit gleichzeitiger Cholinesterasehemmung gewählt, da es eine längere Wirkungsdauer und höhere Augeninnendrucksenkung als andere Miotika, z.B. Pilocarpin, aufweist und das Carbachol-DPE-Kombinationspräparat (insbesondere bei Anwendung der Suspensionstechnologie) damit eine prinzipiell höhere Wahrscheinlichkeit zur Reduzierung der Applikationshäufigkeit auf 2 x täglich bei konstanter Augeninnendrucksenkung gewährleistet.

Zu den Vorteilen von Carbachol bei Kombinationspräparaten (vergleiche PCT/EP 91/01295).

Das erfindungsgemäße Kombinationspräparat besteht aus der Kombination der folgenden Wirkstoffgruppen:

### Kombinationspräparat I

Direktes Parasympathomimetikum mit gleichzeitiger Cholinesterasehemmung/Direktes Sympthomimetikum
Carbachol (Lösung oder Suspension) 3 % oder weniger und DPE (Dipivalylepinephrin 0,1 %)

### Beispiel

(A) Zusammensetzung einer Lösung:
   Carbachol-Augentropfen 3 %
   Dipivalylepinephrin 0,1 %
   Konservierungsmittel: Benzalkoniumchlorid.
(B) Zusammensetzung einer Suspension:
   Carbachol-Augentropfen 3 %
   Dipivalylepinephrin 0,1 %
   Konservierungsmittel: Benzalkoniumchlorid.

### Kombinationspräparat II:

Direktes Sympathomimetikum/Direktes Sympatholytikum Dipivalylepinephrin 0,1 % oder weniger (Untergrenze 0,09 %) und Betablocker, z.B. Betaxolol-S 0,28 % oder nicht selektiver Beta-1-Beta-2-Blocker (Suspension)
oder:
Dipivalylepinephrin 0,1 % oder weniger (Untergrenze 0,09 %)und Betablocker, z.B. Betaxolol 0,5 % (Lösung) oder nicht selektiver Beta-1-Beta-2-Blocker.

### Beispiele:

Dipivalylepinephrin 0,09 %
mit Betaxolol-Suspension 0,28 %
bzw. Betaxolol-Lösung 0,5 %.

Dipivalylepinephrin 0,1 % (Untergrenze: 0,09 %) mit wahlweise folgenden Betablockern als Lösung:
- Befunolol 0,5 % bzw. 0,25 % als Untergrenze
- Levobunolol 0,5 % bzw. 0,25 % als Untergrenze
- Pindolol 0,5 % bzw. 0,25 % als Untergrenze
- Timolol 0,5 bzw. 0,25 % als Untergrenze
- Carteolol 2 % bzw. 1 % als Untergrenze
- Metipranolol 0,3 % bzw. 0,1 % als Lösung
Bei Anwendung der Suspensionstechnologie kann bei gleicher Drucksenkung die Konzentration in den genannten Grenzen reduziert, maximal halbiert werden.

Die zwei Kombinationspräparate auf DPE-Basis können als Lösung oder in Suspensionstechnologie hergestellt werden. Die Suspensionstechnologie in einem Kombinationspräparat führt für beide Substanzen (Komponenten) zu einer besseren Penetration und langsameren Wirkstoffabgabe (Bolus-Charakter der Medikamente entfällt) und damit wiederum zu einer höheren Bioverfügbarkeit mit höherer Wahrscheinlichkeit der konstanten Augeninnendrucksenkung bei nur noch 2 x täglicher Applikation, was eine Reduzierung der Konzentration der Einzelsubstanzen ermöglicht, ohne Einschränkung der Wirkungsdauer und Wirkungsstärke im Vergleich zur freien Kombination der Monosubstanzen.

Beim Kombinationspräparat II, das eine Betablockerkomponente enthält, ergibt sich durch die betablockerbedingte Verminderung der Kammerwasserproduktion eine geänderte Pharmakokinetik und damit eine längere Bioverfügbarkeit der DPE-Komponente, die es möglicherweise erlaubt, die Konzentration der DPE-Komponente oder beider Komponenten bei gleicher Wirkungsdauer und Wirkungsstärke zu reduzieren bei nur 2 x täglicher Applikation des Kombinationspröparates. Das Kombinationspräparat II ist prinzipiell mit einem Beta-1-selektiven (theoretisch besser geeignet), als auch mit allen nicht selektiven Beta-1-Beta-2-Blockern herstellbar. Die Wahl des Betablockers richtet sich danach, mit welchem der am Auge zugelassenen Betablocker, zusammen mit DPE hinsichtlich Stabilität und pH-Wert das beste Ergebnis bei der fixen Kombination erreicht wird (der pH-Wert ist für Penetration und lokale Verträglichkeit entscheidend). Mit nicht selektiven Betablockern läßt sich beim Menschen bei freier Kombination nicht selektiver Betablocker mit DPE eine gute additive den Augeninnendruck senkende Wirkung erreichen, so daß die praktische Relevanz der Verwendung eines Beta-1-selektiven Betablockers bei einem DPE-Betablocker-Kombinationspräparat bisher am Menschen nicht abschließend beantwortet werden kann. Entscheidend ist, daß als Adrenalinkomponente ausschließlich das Prodrug des Adrenalins, das DPE verwendet wird, da es die geringeren lokalen und systemischen Nebenwirkungen im Vergleich zu anderen Adrenalinpräparaten bei gleicher Augeninnendurcksenkung aufweist.

Da im Präparat II ein die Kammerwasserproduktion hemmendes und ein den Kammerwasserabfluß verbesserndes Medikament kombiniert werden, tritt eine Änderung der Pharmakokinetik ein, so daß die Konzentration von DPE von 0,1 % auf 0,09 % gesenkt werden kann, bei vergleichbarer drucksenkender Wirkung.

Die fixe Kombination der Einzelkomponenten in Kombinationspräparaten hat eine längere Wirkungsdauer und höhere Drucksenkung zur Folge als die freie Kombination der Einzelsubstanzen. Der Wirkungsmechanismus der Kombinationspräparate scheint somit nicht nur die Summe der Einzelmechanismen der Monopräparate zu sein - ein überraschendes Phänomen, für das es bisher noch keine gesicherte pharmakologische Erklärung gibt. Die verlängerte und höhere drucksenkende Wirkung der Kombinationspräparate läßt sich nicht nur durch eine Reduzierung des gegenseitigen Auswascheffektes erklären, sondern läßt auch eine Änderung der Pharmakokinetik und/oder eine andere Interaktion der Medikamente im Auge vermuten. Bei Anwendung der Suspensionstechnologie läßt sich eine Reduzierung der Wirkstoffgesamtmenge erreichen, wodurch sich ein höheres Sicherheitsprofil hinsichtlich systemischer und lokaler Nebenwirkungen ergibt. Diese Suspensionstechnologie besteht aus Poly(Styrol-Divinylbenzol)Sulfonsäure (Amberlite IRP-69) 2,5 mg als Wirkstoffträger und Carbomer 934 P 2 mg zur Erhöhung der Depotfunktion. Inwieweit sich die Wirkstoffmenge unter Beibehaltung der 2 x täglichen Applikation bei gleicher Augeninnendrucksenkung reduzieren läßt, muß tierexperimentell für die einzelnen Kombinationspräparate I und II geprüft werden, wobei die Reduzierung der Wirkstoffkonzentration bei der Betablockerkomponente von 0,5 % bei der Lösung auf 0,28 % bei der Suspension bei gleicher drucksenkender Wirkung gesichert ist.

Ein entscheidender Vorteil der erfindungsgemäßen Kombinationspräparate ist für die wässerige Lösung wie auch für die Suspension die nur 2 x tägliche Anwendung. Dies führt zu einem einfacheren Therapieschema und damit zu einer Verbesserung der Compliance bei höherer Wirkungsdauer und stärkerer Drucksenkung als die freie Kombination der Einzelsubstanzen. Beim Carbachol-DPE-Kombinationsporäparat ergibt sich eine Reduzierung der Applikationshäufigkeit bei gleicher drucksenkender Wirkung von Carbachol 3 % (3 x täglich) und Betablocker (2 x täglich) auf eine 2 x tägliche Applikation des Kombinationspräparates. Carbachol-Kombinationspräparate sind die einzigen Miotika-Kombinationspräparate, die bei Zweimalapplikation eine konstante Augeninnendrucksenkung auch bei höheren Augeninnendruckwerten erwarten lassen. Die Doppelwirkung des Carbachols als direktes Parasympathomimetikum und als Cholinesterasehemmer ist daher vorteilhaft in fixer Kombination mit Betablockern (PCT/EP 91/01295), als auch in fixer Kombination mit Dipivalylepinephrin (DPE). Beim Carbachol-DPE-Kombinationspräparat wirken beide Komponenten bei unterschiedlichem Angriffsort abflußverbessernd, was der Pathogenese der Augeninnendruckerhöhung (Abflußverschlechterung) unmittelbar entgegenwirkt.

Die Pharmakokinetik des Carbachol ist es, die diese alte Substanz als Bestandteil einer Miotikum DPE-Kombination im Rahmen eines Kombinationspräparates mit nun nur noch 2 x täglicher Anwendung interessant macht und deutliche Vorteile gegenüber der Pilocarpin-Betablocker-Kombination, z.B. Normoglaucon® oder der Pilocarpin-DPE-Kombination, z.B. Thiloadren® (Applikationshäufigkeit. 3 x täglich, wegen der kurzen Wirkungsdauer der Pilocarpin-Komponente im Vergleich zu Carbachol 3 %) aufweist. Beim erfindungsgemäßen Kombinationspräparat I ist DPE in einer Kombination von 0,1 % oder weniger, bei fixer Kombination mit Carbachol 3 % auch bei engem Kammerwinkel anwendbar, ohne daß zuvor operativ eine periphere Iridektomie angelegt werden muß.

Die neuen Kombinationspräparate können die Stufenleiter einer compliancegerechten medikamentösen Kombinationstherapie verlängern und sind in Anbetracht der steigenden Lebenserwartung der Bevölkerung eine medizinische und ethische Notwendigkeit.

Die Konzentration an Dipivalylepinephrin in dem erfindungsgemäßen Präparat beträgt vorzugsweise 0,1 bis 0,09 %, die Konzentration des Carbachols 3 bis 1,5 % und die des Betablockers 0,5 bis 0,25 %.

## Patentansprüche

1. Präparat zur Senkung des Augeninnendrucks auf der Basis von Dipivalylepinephrin, dadurch **gekennzeichnet,** daß es zusätzlich Carbachol oder einen Beta-Blocker enthält.

2. Präparat nach Anspruch 1, dadurch **gekennzeichnet,** daß das Dipivalylepinephrin in einer Konzentration von 0,1 % bis 0,09 % vorliegt.

3. Präparat nach Anspruch 2, dadurch **gekennzeichnet,** daß das Carbachol in einer Konzentration von 3 % bis 1,5 % vorliegt.

4. Präparat nach Anspruch 2, dadurch **gekennzeichnet,** daß der Betablocker als Beta-1-Beta-2-Blocker oder selektiver Beta-1-Blocker in einer Konzentration von 0,5 % bis 0,25 % vorliegt.

5. Kombinationspräparate nach Anspruch 1 bis 4, dadurch **gekennzeichnet,** daß sie in Form von Augentropfen (Lösung oder Suspension), Augensalbe, Augen-Gel, Augeninsert oder auf einem sonstigen Medikamententräger zur lokalen Applikation am Auge zubereitet sein können.
